# EUROPEAN PATENT SPECIFICATION

(11) **EP 2 175 800 B1**
(45) Date of publication and mention of the grant of the patent: **11.12.2019**
(21) Application number: 08771718.7
(22) Date of filing: 23.06.2008
(51) Int. Cl.: A61F 2/02, A61B 17/11, A61B 17/06, A61L 27/24, A61F 2/00, A61F 2/08, A61B 17/04

(54) **COMPOSITE IMPLANT FOR SURGICAL REPAIR**
KOMPOSIT-IMPLANTAT FÜR DIE CHIRURGISCHE REPARATUR
IMPLANT COMPOSITE POUR PRÉPARATION CHIRURGICALE

(30) Priority: 13.07.2007 US 777733
(43) Date of publication of application: 21.04.2010
(73) Proprietor: Synovis Orthopedic and Woundcare, Inc., St Paul MN 55114 (US)
(72) Inventor: BRUNELLE, John, Laguna Beach, California 92651-1570 (US); SIEGEL, Joshua, Exeter, New Hampshire 03833 (US); NGUYEN, Christine, Glendora, California 91741 (US); YANTZER, Brenda, Gainesville, Florida 32606 (US); SANDER, Thomas, Gainesville, Florida 32653 (US)
(74) Representative: Boult Wade Tennant LLP
(86) International application number: PCT/US2008/067861
(87) International publication number: WO 2009/012021

(56) References cited:
- EP-A1- 0 734 736
- EP-A1- 1 177 800
- WO-A1-2007/005394
- DE-U1- 8 813 452
- US-A- 4 979 956
- US-A1- 2007 112 360
- US-B1- 6 478 825
- US-B1- 6 946 003
- US-B2- 6 626 950
- US-B2- 6 991 652

## Description

### Background

Surgical repair of damaged soft tissue is a procedure that is being carried out with increasing frequency. The simplest method for many soft tissue repairs is to suture together the torn or damaged portions of the affected tissue. This relatively simple method carries several drawbacks, however. For instance, the recovery period following a procedure is extremely long and often includes the development of a large amount of scar tissue that can lead to permanent loss of strength, range of motion, etc.

More recent advances have led to the development of tissue augmentation materials that can be affixed to the damaged and/or surrounding tissues to facilitate healing. For instance, permanent implants can be used to replace damaged or missing natural tissues. Other procedures utilize scaffolding-type implants that can stabilize the damaged tissue while also providing a framework to encourage natural re-growth and repair of damaged tissue.

Problems still exist with such procedures, however. For instance, the term permanent' with regard to biological implants is relative, and permanent implants will often require replacement during the recipient's lifetime. Scaffolding materials, while showing great potential with regard to encouraging long-term repair and recovery of damaged tissue, can be relatively delicate and can present both handling and placement difficulties during surgical procedures. In addition, scaffolding materials generally offer little in the way of mechanical strength to the damaged tissues in the short term, i.e., immediately following implantation and prior to the regeneration of new, stronger natural tissue.

Accordingly, what is needed in the art are implantable materials that can exhibit strength and tenacity so as to provide improved handling during surgical procedures as well as mechanical reinforcement of the repair site upon introduction thereto, while also exhibiting the desirable characteristics of a scaffolding material so as to direct and support the long-term regeneration and repair of the natural tissues. US4,979,956 relates to a device and method for tendon and ligament repair.

In one embodiment, the disclosed subject matter is directed to a biocompatible implant according to claim 1.

In one embodiment, the elongated member can have a greater tensile strength than does the scaffold. According to one embodiment, a longitudinal load placed upon the implant along an axis of the elongated member can be borne primarily by the elongated member.

The scaffold comprises collagen. For example, a scaffold can contain crosslinked collagen.

In one preferred embodiment, an elongated member can be a suture, but this is not a requirement of the disclosed implants. Other suitable materials for use as elongated members can include, e.g., non-pliable members, polymer fabrics, and elongated members derived from natural tissues such as ligaments, tendons, and the like.

An implant can include additional materials as well. For instance, an implant can include biologically active materials such as growth factors, antibiotics, living cells, etc., as well as structural materials including anchoring materials, additional scaffolds, additional implants, and so on.

Implants as disclosed herein can be delivered to tissues in need thereof such as damaged tendons, ligaments, and the like. Disclosed implants can be utilized to fill soft tissue defects, for instance in cosmetic and reconstructive surgery as well as a suture bolster, among other uses. For example, an elongated member can be utilized to manipulate and locate a scaffold at a repair site, to apply the implant with a desired tension at a site, as well as to attach an implant at the damaged site.

### Brief Description of the Figures

A full and enabling disclosure of the present subject matter, including the best mode thereof, to one of ordinary skill in the art, is set forth more particularly in the remainder of the specification, including reference to the accompanying figures, in which:
FIG. 1 is one embodiment of a composite implant as described herein;
FIG. 2 is another embodiment of a composite implant as described herein;
FIGS. 3A-3D illustrate embodiments of composite implants as described herein including reinforcement materials incorporated with the implants;
FIG. 4A-4E illustrate a collagen-containing scaffold material (FIG. 4A), a composite implant as described herein including the scaffold material of FIG. 4A (FIG. 4B), and the formation steps for forming a locking stitch as may be utilized in forming a composite implant as described herein (FIGS. 4C-4E).
FIG. 5A illustrates a scaffold as may be used in forming a composite implant that has been pre-treated to include fenestrations or perforations at predetermined positions;
FIG. 5B illustrates a composite implant as described herein including the scaffold material of FIG. 5A;
FIG. 5C illustrates another embodiment of a composite implant as described herein;
FIG. 6 illustrates an embodiment of a composite implant as described herein including two scaffold sections following initial formation (FIG. 6A) and after forming the composite to the implant shape (FIG. 6B);
FIGS. 7A illustrates another embodiment of a composite implant as described herein;
FIG. 8 illustrates a reference example of a composite implant as described herein;
FIG. 9A illustrates a formation method for the composite implant illustrated in FIG. 9B;
FIGS. 9C and 9D illustrate additional embodiments of composite implants as described herein;
FIGS. 10A-10D illustrate one embodiment of a formation method for a composite implant as described herein;
FIGS. 11A-11D illustrate a delivery method as may be used during a surgical repair procedure for delivering a composite implant as described herein to a damaged soft tissue site; and
FIGS. 12A-12F illustrate a delivery method as may be used during a surgical repair procedure for delivering a composite implant as described herein to a damaged soft tissue site.

Repeat use of reference characters in the present specification and drawings is intended to represent the same or analogous features or elements of the present disclosure.

### Detailed Description

Reference will now be made in detail to various embodiments of the disclosed subject matter, one or more examples of which are set forth below. Each embodiment is provided by way of explanation of the disclosed subject matter, not limitation thereof. For instance, features illustrated or described as part of one embodiment, may be used with another embodiment to yield a still further embodiment.

In general, the presently disclosed subject matter is directed to biocompatible implants, methods for forming the implants, and methods for using the implants. Biocompatible implants as described herein include at least two materials that have been combined in a fashion so as to maintain the desirable characteristics of each component. For example, disclosed composite implants can provide the maneuverability, strength, tenacity, and/ or immediate reinforcement ability of suture-type materials combined with the tissue regeneration and excellent long-term healing characteristics of scaffold materials. In one embodiment, the disclosed composite implants can be utilized in surgical repair procedures for damaged human or animal soft tissues such as, e.g., tendons and ligaments. In other embodiments, the disclosed materials can be used in procedures directed to other tissues including muscles, vascular tissue, synovial tissue, biomembranes such as endocranium, pericardium, pleura, organs, bones, and the like. For example, disclosed materials can be utilized as suture bolsters for damaged organs such as damaged connective, lung or liver tissue as well as other uses described further below.

Included as a component of the disclosed implants can be one or more scaffolding materials. As utilized herein, the term 'scaffold' can generally refer to biocompatible materials that can facilitate cellular growth and development when located in proximity to living cells. Scaffold materials encompassed herein include those designed for in vivo, ex vivo, and/or in vitro use. In general, scaffold materials can describe a physical structure that can allow cellular ingrowth to the scaffold. For example, a scaffold can include macro- and/or microporosity that can allow cellular propagation throughout all or a portion of the scaffold. In one embodiment, a scaffold can include a matrix with a mesh size, ξ, or a pore size, ρ, that can allow cellular propagation and/or ingrowth throughout the matrix.

The scaffold comprises collagen. Scaffolds encompassed by the disclosed subject matter can include one or more materials that encourage the growth and development of a cellular construct. For instance, a scaffold can further include one or more synthetic or natural biocompatible polymers that have been shown to promote wound healing. Biocompatible synthetic polymers as may be utilized in forming a scaffold can include, e.g., polyurethanes, polyesters, polyethylenes, silicones, polyglycolic acid (PGA), polylactic acid (PLA), copolymers of lactic and glycolic acids (PLGA), polyanhydrides, polyorthoesters, and the like. A scaffold can further include one or more natural polymers including, e.g., chitosan and glycosaminoglycans.

In one embodiment, a scaffold can include or be formed entirely of a hydrogel matrix. Hydrogel scaffolds are known in the art and are generally defined to include polymeric matrices that can be highly hydrated while maintaining structural stability. Suitable hydrogel scaffolds can include non-crosslinked and crosslinked hydrogels. In addition, crosslinked hydrogel scaffolds can optionally include hydrolyzable portions, such that the scaffold can be degradable when utilized in an aqueous environment. For example, in one embodiment, a scaffold can include a cross-linked hydrogel including a hydrolyzable cross-linking agent, such as polylactic acid, and can be degradable in an aqueous environment.

Hydrogel scaffolds can include natural polymers such as glycosaminoglycans, polysaccharides, proteins, and the like, as well as synthetic polymers, as are generally known in the art. A non-limiting list of polymeric materials that can be utilized in forming hydrogel scaffolds can include dextran, hyaluronic acid, chitin, heparin, collagen, elastin, keratin, albumin, polymers and copolymers of lactic acid, glycolic acid, carboxymethyl cellulose, polyacrylates, polymethacrylates, epoxides, silicones, polyols such as polypropylene glycol, polyvinyl alcohol and polyethylene glycol and their derivatives, alginates such as sodium alginate or crosslinked alginate gum, polycaprolactone, polyanhydride, pectin, gelatin, crosslinked proteins peptides and polysaccharides, and the like.

Hydrogel scaffolds can be formed according to any method as is generally known in the art. For instance, a hydrogel can self-assemble upon mere contact of the various components or upon contact in conjunction with the presence of particular external conditions (such as temperature or pH). Alternatively, assembly can be induced according to any known method following mixing of the components. For example, step-wise or chain polymerization of multifunctional monomers or macromers can be induced via photopolymerization, temperature dependent polymerization, and/or chemically activated polymerization. Optionally, a hydrogel can be polymerized in the presence of an initiator. For example, in one embodiment, a hydrogel scaffold can be photopolymerized in the presence of a suitable initiator such as Irgacure® or Darocur® photoinitiators available from Ciba Specialty Chemicals. In another embodiment, a cationic initiator can be present. For example, a polyvalent elemental cation such as Ca<2+>, Mg<2+>, Al<3+>, La<3+>, or Mn<2+> can be used. In another embodiment, a polycationic polypeptide such as polylysine or polyarginine can be utilized as an initiator.

The scaffold contains collagen. Collagen is the most abundant fibrous structural protein found in mammals and has been shown to exhibit many desirable qualities in scaffolding materials. For example, in addition to good bioaffinity and histocompatibility, wound healing cells such as fibroblasts have been shown to have good affinity for collagen, and the presence of collagen in a scaffold can encourage and promote cell growth and differentiation of the tissues/cells associated with the scaffold.

Collagen encompassed by the present disclosure can include any collagen type or combination of collagen types. For instance, a collagen-containing scaffold can include any one or combination of the currently known 28 types of collagen. Typically, a collagen-containing scaffold can include at least some type I and/or type II collagen, but this is merely due to the fact that types I and II collagen are the most abundant types of collagen, and it should be understood that the presence of either of these types is not a requirement in a collagen-containing scaffold as disclosed herein.

A collagen-containing scaffold can be derived of any suitable collagen source and formed according to any suitable method as is understood by one of ordinary skill in the art. For example, a collagen-based scaffold can include natural collagen-containing tissues that can be allograft, autograft, and/or xenograft tissues. Natural collagen-containing tissues that can be used to form a scaffold can include, without limitation, soft tissues including ligament, tendon, muscle, dura, pericardium, fascia, peritoneum, and the like and can be derived from any host source (human, equine, porcine, bovine, etc.).

A natural tissue scaffold can be processed to remove some or all of the cellular components of the tissue. For example, a tissue for use as a scaffold can be air-dried or lyophilized to kill cells contained therein. Thermal shock, sonication or ultrasound treatment, changes in pH, osmotic shock, mechanical disruption, or addition of toxins can also induce cell death or apoptosis. Other treatments to de-cellularize or denature the tissue are possible using radiation, detergents (e.g., sodium dodecyl sulfate (SDS)), enzymes (RNAase, DNAase), or solvents (alcohol, acetone, or chloroform). These techniques are only some of the examples of techniques to de-cellularize, denature or chemically modify all or part of the tissue and are not meant to limit the scope of the disclosure. For example, methods of de-cellularizing can utilize, for example, enzymes such as lipases combined with other enzymes and, optionally, detergents. Treatment with hypotonic and/or hypertonic solutions, which have non-physiological ionic strengths, can promote the de-cellularization process. These various de-cellularization solutions generally are suitable as treatment solutions. Proteases also can be used effectively to de-cellularize tissue. The de-cellularization can be performed in stages with some or all of the stages involving differential treatments. For example, a potent mixture of proteases, nucleases and phospholipases could be used in high concentrations to de-cellularize a tissue.

Collagen-containing materials can be processed according to any suitable methods during a scaffold preparation process. For instance, a collagen-containing scaffold can be derived from reconstituted collagen. The capability of utilizing reconstituted collagen to form a scaffolding material was first published by Bell, et al. in 1979 (Proc. Natn. Acad. Sci. USA, 76, 1274-1278). In general, methods for forming scaffolds from reconstituted collagen include extraction and purification of collagen(s) from connective tissues by solubilization that can be acidic, alkaline, neutral and/or enzymatic in nature. The extracted collagen can be broken down to monomeric and/or oligomeric level and stored as a powder or liquid. Upon rehydration, a solution can form that can be molded and crosslinked via chemical or physical methods to form a scaffold.

Variations and improvements upon initially-disclosed processes have been disclosed. For example, U.S. Pat. No. 6,623,963 to Muller, et al., describes a method for forming a scaffold that includes solubilizing animal cartilage tissue by physical and/or chemical treatment processes that include treatment with various buffers to remove impurities and to separate the solid and liquid phases; physical treatment to separate solid and liquid phases, such as by centrifugation; and treatment with a proteolytic enzyme that breaks the crosslinking of the collagen in its telopeptide region into its virtually non-crosslinked, atelocollagen, triple helix form. The collagen thus obtained is then reconstituted, i.e., the non-crosslinked, atelocollagen form of collagen reestablishes its crosslinking between the variable regions along the collagen molecule, including some remaining residues in the telopeptide region. As a result, the solubilized collagen loses its liquid or gel-like consistency and becomes more rigid with a higher degree of structural integrity such that it may be utilized as a scaffold.

U.S. Pat. No. 4,488,911 to Luck et al., describes the formation of collagen fibers free of the immunogenic, telopeptide portion of native collagen. The telopeptide region provides points of crosslinking in native collagen. The fibers, which may be crosslinked, are described for use as sponges, prosthetic devices, films, membranes, and sutures. In the method described in the '911 patent, (non-Type II; Type I and others) collagen obtained from tendons, skin, and connective tissue of animals, such as a cow, is dispersed in an acetic acid solution, passed through a meat chopper, treated with pepsin to cleave the telopeptides and solubilize the collagen, precipitated, dialyzed, crosslinked by addition of formaldehyde, sterilized, and lyophilized. The '911 patent indicates that its disclosed method obtains the atelocollagen form of collagen, free from non-collagen proteins, such as glycosaminoglycans and lipids. Further, the collagen may be used as a gel to make, for example, a membrane, film, or sponge and the degree of crosslinking of the collagen can be controlled to alter its structural properties.

Of course, the above described methods are merely embodiments of processing as may be carried out in forming a collagen-containing scaffold as may be utilized in forming the disclosed composite implants and the present disclosure is in no way limited to these embodiments. Many other processing methods and scaffolds formed thereby are known to those of ordinary skill in the art and thus are not described at length herein, any of which may be utilized according to the disclosure.

A scaffold may be processed as desired prior to forming a composite implant. For instance, a natural or reconstituted tissue can be stabilized through crosslinking. Generally, a stabilization process operates by blocking reactive molecules on the surface of and within the scaffold, thereby rendering it substantially non-antigenic and suitable for implantation. In 1968, Nimni et al. demonstrated that collagenous materials can be stabilized by treating them with aldehydes. (Nimni et al., J. Biol. Chem. 243:1457-1466 (1968).) Later, Various aldehydes were tested and glutaraldehyde was shown to be capable of retarding degeneration of collagenous tissue. (Nimni et al., J. Biomed. Mater. Res. 21:741-771 (1987); Woodroof, E. A., J. Bioeng. 2:1 (1978).) Thus, according to one embodiment, a glutaraldehyde stabilization process as is generally known in the art may be utilized in forming a scaffold (see, e.g., U.S. Pat. No. 5,104,405 to Nimni).

A glutaraldehyde process is only one processing method, however, and a scaffold material processed according to any other method as is known in the art may alternatively be utilized. For example, a scaffold material as may be utilized in a disclosed composite implant can be stabilized according to a physical crosslinking process including, without limitation, radiation treatment, thermal treatment, electron beam treatment, UV crosslinking, and the like.

In one preferred embodiment, a scaffold can be processed according to a non-glutaraldehyde crosslinking process. For example, non-glutaraldehyde crosslinking methods as disclosed in U.S. Pat. Nos. 5,447,536 and 5,733,339 to Girardot, et al. can be utilized. According to one such embodiment, a collagen-containing scaffold can be crosslinked via formation of amide linkages between and within the molecules of the scaffold. For instance, di- or tri-carboxylic acids and di-or tri-amines of about six to eight carbon atoms in length can be used in a sequential manner to form amide crosslinks.

Optionally, a scaffold can be formed to include additional materials. For instance, cellular materials can be retained in or loaded into a scaffold. For example, chondrocytes and/or fibroblasts can be retained in a natural tissue scaffold or loaded into a scaffold prior to implantation. In one embodiment, a scaffold can be seeded with cells through absorption and cellular migration, optionally coupled with application of pressure through simple stirring, pulsatile perfusion methods or application of centrifugal force. In general, cell seeding can usually be carried out following combination of a scaffold with the other components of the implant, described in more detail below, to form a composite implant as described herein.

Other materials as may be incorporated into the disclosed composite implants via the scaffold can include any other additive as is generally known in the art. For instance, biologically active agents such as growth factors, antibiotics, extra cellular matrix components, or any other chemical or biological agent as may be beneficially incorporated into a scaffold is encompassed by the presently disclosed subject matter. Additional materials can be loaded into a scaffold, applied to a surface of a scaffold, or combined with another component of an implant, as desired.

In forming a composite implant, a scaffold can be combined with an elongated member that can bring desirable characteristics to the composite including one or more mechanical characteristics such as strength, tenacity, load distribution and maneuverability. Beneficially, an elongated member can be affixed to a scaffold so as to provide a means for manipulating and locating a scaffold at a desired location.

In one embodiment, an elongated member can also bear the majority of a longitudinal load under which the composite may be placed. For instance, during a surgical procedure, an implantable composite as described herein can be placed at a repair site and an elongated member of the composite can be used to locate the composite at the repair site, and, in one embodiment, also bear the majority of any longitudinal load under which the composite is placed during the procedure. For example, a composite can be pulled to the desired repair site through and/or around existing tissues and the scaffold can be aligned as desired at the target location without fear of damage to the scaffold, as the elongated member of the scaffold is utilized to locate the implant as desired. Thus, in certain embodiments, forces placed upon an implant during and/ or following location of the implant at a repair site can be primarily borne by the elongated member and the scaffold can be mechanically isolated from such forces.

In one embodiment, materials for use as an elongated member can have a tensile strength (i.e., the longitudinal stress required to rupture the elongated member) greater than that of the scaffold. For instance, an elongated member can exhibit a tensile strength of at least about 1 N, or greater than about 3000 N, in another embodiment.

Elongated members can have any cross sectional geometry, e.g., round, square, rectangular, toroid, complex geometric cross-sections, such as a multi-nodular cross sections, and the like, and can generally have an aspect ratio (length/effective diameter) of at least about 10. Elongated members can be formed of natural materials, synthetic materials, or some combination thereof.

In one embodiment, elongated members can be fibrous materials. For instance, elongated members can be mono- or multi-filament materials. Moreover, the term encompasses single or multi-component materials. For instance, an elongated member can include multi-component fibers including core/sheath fibers, islands-in-the-sea fibers, and so on, as well as members including adjacent lengths of different materials. Elongated members can also incorporate a plurality of fibrous materials. For instance, an elongated member can include a fabric (e.g., a woven, knit, or nonwoven textile or mesh material) that can partially or completely cover a scaffold.

In one preferred embodiment, an elongated member can be formed of a suture material. Any suture material as is known in the art can be utilized, with the preferred suture material generally depending upon the nature of the repair for which the composite implant is to be utilized. Suture material for an implantable composite can be absorbable or non-absorbable, as desired. Suture can be of any size (e.g., from #11-0 up to #5 in size), suture can be multifilament and braided or twisted, or can be mono-filament. Suture can be sterile or non-sterile, of natural, synthetic, or a combination of materials. In one embodiment, suture material can be coated. Typical coatings can include, for example, collagen, magnesium stearate, PTFE, silicone, polybutilate, and antimicrobial substances.

A large variety of suitable suture is known to those of skill in the art and can include, without limitation, collagen, catgut, polyglycolic acid, polyglactin 910, poliglecaprone 25, polydioxanone, surgical silk, surgical cotton, nylon, polybutester, polyester fibers, polyethylene fibers, polypropylene fibers, and the like. For instance, polyethylene suture such as co-braided polyethylene suture can be utilized in one embodiment.

Elongated members of the disclosed implantable composites are not limited to suture materials, however, and the term 'elongated member' is intended to encompass any materials having an overall aspect ratio (L/D) greater than about two that can be combined with one or more scaffolds for formation of an implantable composite as described herein. For example, in one embodiment, an elongated member can comprise a natural connective tissue such as a ligament or tendon that can be affixed to a scaffold material so as to provide maneuverability, strength and/or tenacity to the composite structure.

In any case, one or more elongated members can be affixed to a scaffold so as to form an implantable composite. In particular, an elongated member can be affixed to a scaffold such that the two are held in contact with one another over a length of a scaffold surface. For instance, in one embodiment, the two can be held in contact with one another over a length that extends from one edge of a scaffold to an opposite edge of the scaffold as measured across a surface of the scaffold.

In another embodiment, in addition to being held in contact with one another along a length of a surface of the scaffold, the materials can be combined such that a longitudinal load placed upon the composite can be effectively translated to and primarily borne by the elongated member and the scaffold can be mechanically isolated and protected from damage, misalignment, and the like during and following implantation.

In one embodiment, an elongated member can provide mechanical reinforcement to a surgical site. For instance, an elongated member can reinforce damaged tissue at a surgical site prior to and during generation of new tissue while the new tissue generation itself can be directed and encouraged due to the presence of the scaffold.

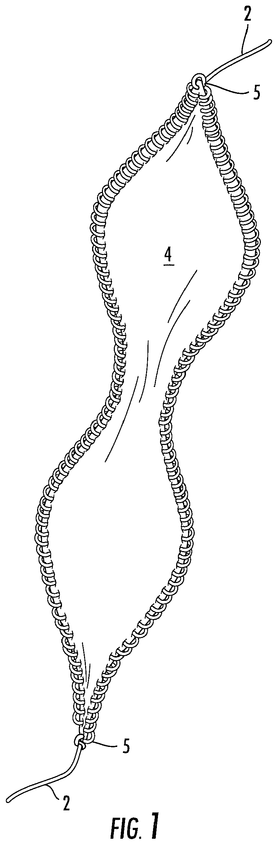

Referring to Fig. 1, one embodiment of a composite implant is illustrated. The composite includes a scaffold 4 in combination with two lengths of suture 2, each length 2 being stitched along an edge of scaffold 4 with a reinforcing stitch, as shown. Scaffold 4 can be preformed to any desired size and shape. For instance, the scaffold 4 of Fig. 1 includes a central area of a smaller cross sectional area than the adjacent sections. Such a geometric configuration can be used to, e.g., properly locate the scaffold at a repair site. Scaffold 4 can also be tapered at one or both ends, as shown, to improve the manipulation and maneuverability of the implant during a surgical procedure.

At either end of scaffold 4, suture 2 is affixed to the scaffold with a locking stitch 5. A locking stitch 5 can mechanically isolate the scaffold 4 from longitudinal forces placed upon the implant. More specifically, when a composite is placed under a longitudinal load, for instance during or following placement of the composite at a surgical site, the suture 2 can bear the majority of load, and the scaffold 4 can be protected from damage. Suture 2 can be used to manipulate and locate the implant during surgery. It can also be used to attach the implant to surrounding tissues in the desired fashion, e.g., with suitable tension, freedom of motion, etc.

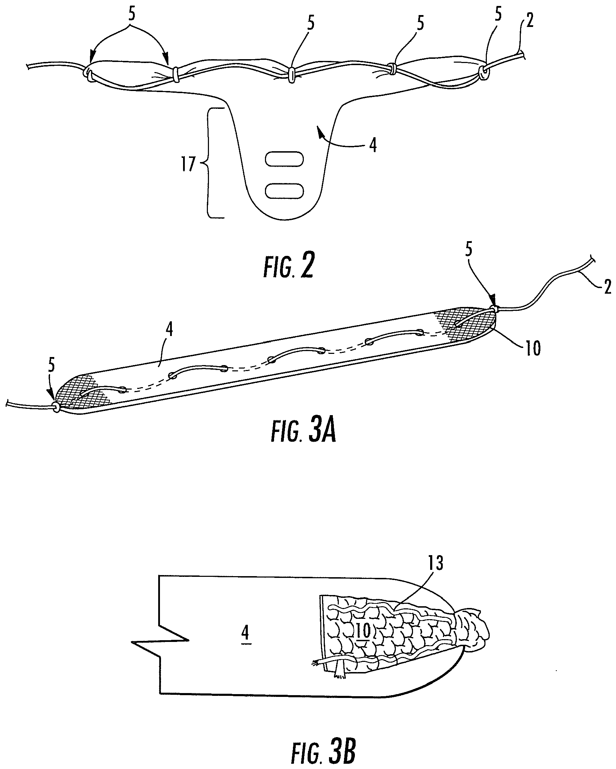

Another embodiment of a composite implant is illustrated in Fig. 2. In this embodiment, scaffold 4 has a design for, e.g., additional tissue augmentation, and includes an extension 17 as shown. As can be seen, suture 2 is affixed to scaffold 4 at a plurality of locations and extends from each end of scaffold 4. At each of the five fixation points, suture 2 is stitched to scaffold 4 with a locking stitch 5. According to this embodiment, should the scaffold 4 be placed under a longitudinal load, the load can translate to the suture 2 at a locking stitch 5. For example, upon placement of a longitudinal load on a scaffold, as during manipulation through or around tissue, a segment of the scaffold may elongate under the applied load, but upon the load reaching a locking stitch 5, the load can translate to the suture 2, and mechanically isolate the scaffold 4 from the load, preventing damage to the scaffold 4.

Fig. 3A and 3B illustrate other embodiments of implantable composites. As can be seen with reference to Fig. 3A, a suture 2 and a scaffold 4 are interwoven across a length of the scaffold 4. At the terminal ends of the scaffold, the scaffold has been reinforced with the addition of a fabric mesh 10. For example, a reinforcing mesh 10 can be affixed to a scaffold 4 with running suture 13 as shown in 3B. Any suitable material can be utilized to reinforce an area of a scaffold. For instance a nonwoven, knit, or woven fabric formed of any suitable biocompatible material can be utilized. A reinforcement material can cover one or more portions of a scaffold, as illustrated in Fig. 3B, or, in one embodiment, can envelope an entire scaffold. For instance, a reinforcement material can cover an end of a scaffold. Optionally, a reinforcement material can be a portion of an elongated member. For instance, a reinforcement material can cover at least a portion of a scaffold, as shown in Figs. 3A and 3B, and a length of fiber that is incorporated into the reinforcement material can extend therefrom to provide the portion of the elongated member that can be utilized in manipulated the composite implant, as discussed further below.

Referring again to Fig. 3A, a suture 2 can be fixed to a scaffold 4 with a locking stitch 5 that passes through both the mesh 10 and the scaffold 4. The scaffold 4 can thus be protected from damage that could otherwise be caused due to longitudinal forces applied to a composite. Interweaving of the suture 2 with the scaffold 4 can provide additional mechanical support to the scaffold 4 and can aid in proper alignment of the scaffold at an implant site, and can also prevent the scaffold from migrating from the suture, even without a force translation mechanism such as a locking stitch 5.

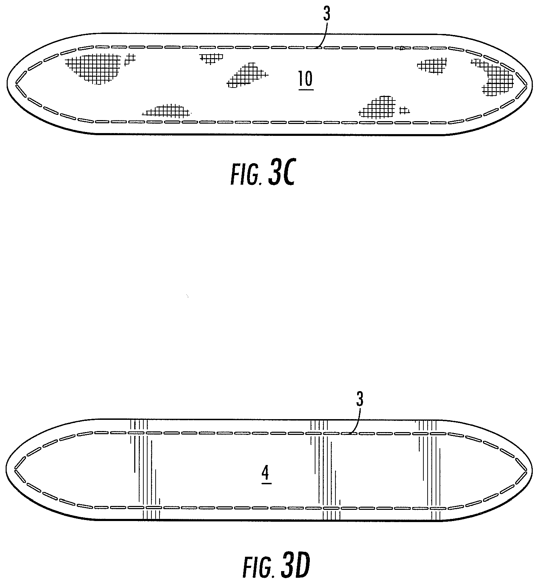

Figures 3C and 3D illustrate another embodiment of a portion of a fabric reinforced composite implant. Figure 3C illustrates a first side of an implant and Figure 3D illustrates the opposite side of the implant. In this particular embodiment, the scaffold 4 is completely covered on the first side (Figure 3C) with a reinforcement fabric 10 that is stitched 3 to the underlying scaffold 4 that is visible in Figure 3D.

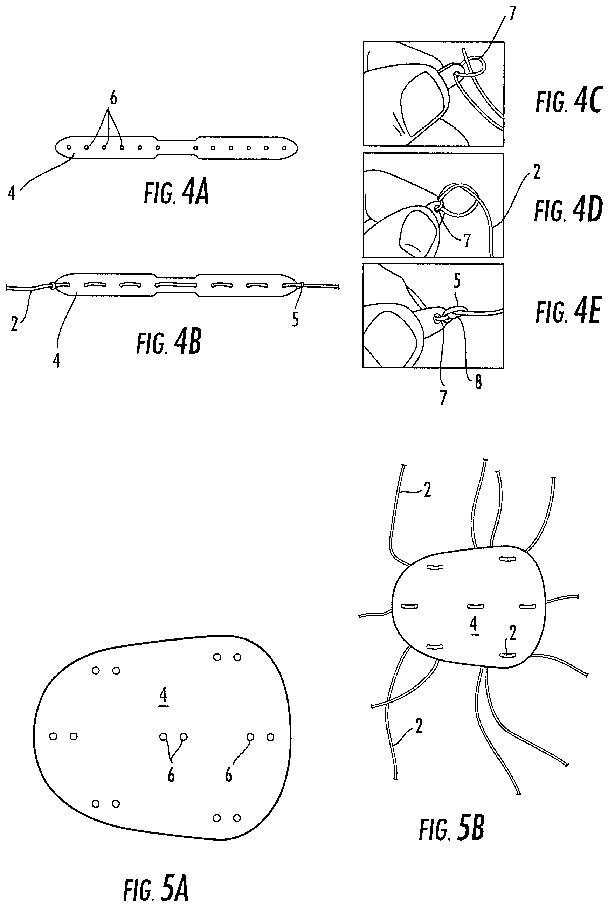

Referring to Fig. 4A, the illustrated scaffold 4 includes a plurality of pre-formed fenestrations and/or perforations 6 that can be used in forming the composite implant. Fenestrations 6 can be formed according to any suitable methods, e.g., mechanical cutting, laser cutting, etc., and of any shape, size, width, length, spacing, vertical or horizontal direction, etc. In one embodiment, fenestrations 6 can be formed at predetermined locations to, for example, provide particular alignment to the composite, to provide particular load-bearing capabilities to the composite (e.g., longitudinal load-bearing in multiple directions, tensile load-bearing, etc.), and so on. FIG. 4B illustrates a composite including the scaffold 4 of FIG. 4A and a length of suture 2 woven through the preformed fenestrations 6 and stitched with a locking stitch 5 at each end of the composite.

FIGS. 4C-4E illustrate one embodiment for forming a locking stitch 5 as may be used to isolate a scaffold 4 from a longitudinal load placed on the composite and protect the scaffold 4 from damage due to the load. In particular, FIG. 4C illustrates a first knot 7 that can fix the scaffold 4 and the suture 2 to one another such that neither can move in relation to the other. FIG. 4D illustrates formation of a second knot 8 that prevents slippage of first knot 7 and isolates the scaffold 4 from a longitudinal load placed on the composite, and FIG. 4E illustrates the completed locking stitch 5.

FIGS. 5A and 5B illustrate another embodiment of a scaffold 4 that has a design for utilization in, e.g., glenoid resurfacing and includes a plurality of preformed fenestrations 6. As can be seen, multiple sutures 2 can be affixed to the scaffold 4, as illustrated in FIG. 5B. According to this embodiment, any or all of the sutures 2 can be used to manipulate the implant. Such an embodiment may be beneficial for properly locating an implant during reconstructive surgery. A large, multi-dimensional scaffold, such as that illustrated in Figure 5A can be more easily, successfully, and accurately located at a surgical site due to isolation of the scaffold 4 from forces applied to the composite during the procedure.

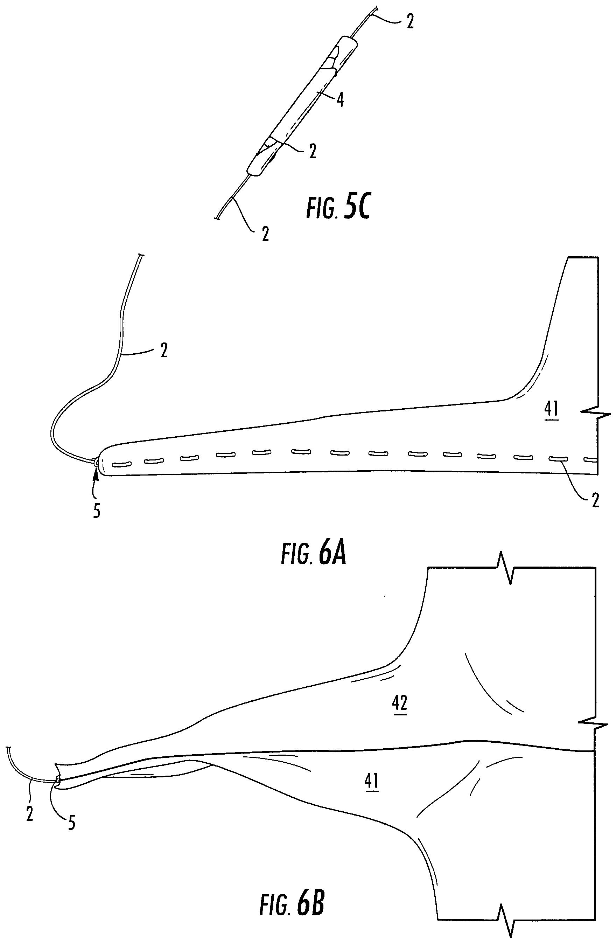

Figure 5C illustrates another embodiment of a composite implant. As can be seen, this implant includes a rolled scaffold 4 and two sutures 2. At either end of the scaffold 4 a suture 2 has been stitched to the scaffold 4 such that the scaffold 4 is held in the desired rolled shape, with a length of suture 2 extending from either end of the scaffold 4, as shown.

Another embodiment of a composite implant as described herein is illustrated in Figs. 6A and 6B. In this particular embodiment, the implant includes two scaffolds 41, 42, which can be the same or different materials, combined with a single elongated member 2. Fig. 6A illustrates the implant during formation. As can be seen, the composite includes a first scaffold 41 and a single suture 2. The suture 2 is woven through an edge of the first scaffold 41 and held with a locking stitch 5 at the illustrated end. Fig. 6B illustrates the composite following completion of the formation process. The complete composite includes two scaffolds 41, 42. The suture 2 has been utilized to combine the two scaffolds 41, 42 together and also, through fixation to the scaffolds with the locking stitch 5, mechanically isolate both of the scaffolds from longitudinal load applied to the composite.

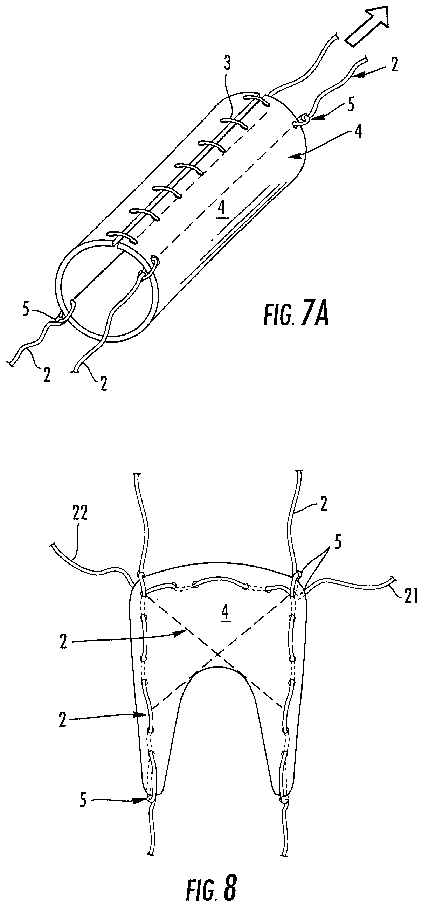

Fig. 7A illustrates another embodiment of a composite implant encompassed by the present disclosure. According to this particular embodiment, one or more elongated members can be affixed to a three dimensional scaffold to form a composite implant. For instance, a scaffold can be formed to a hollow cylindrical shape, as illustrated, for use as, e.g., a vascular graft, nerve wrap, tendon wrap. Suture 3 can hold the composite implant in the desired shape while suture 2 can be affixed to the scaffold and extend from the scaffold for purposes of manipulating the composite. Alternatively, a single length of suture material can be used to maintain the desired shape of the implant as well as extend from the scaffold for purposes of manipulating the composite and protect the scaffold structure from tensile loads. Scaffold materials can be formed into any desired shape through, e.g., folding, rolling, cutting, or any other formation process.

FIG. 8 illustrates yet another embodiment of a composite implant as disclosed herein. As can be seen, this particular embodiment includes a plurality of suture 2 and lengths of suture 21, 22 extending from the scaffold 4 in a variety of directions. Not all of the lengths of suture extend from the scaffold 4 in two opposite directions, however. For instance sutures including the marked extensions 21, 22 extend from scaffold 4 as shown, and are affixed to the scaffold 4 at the point of extension with a locking stitch 5. At the other end of these particular suture lengths, however, the suture lengths are affixed to the scaffold 4, but they do not extend beyond the edge of the scaffold 4 from this end. Nevertheless, sutures including extensions 21, 22 can still be utilized to manipulate, align and/or attach the scaffold 4 during a surgical procedure as well as, in certain embodiments, mechanically isolate the scaffold 4 from a longitudinal load applied along the length of the suture. The sutures can also serve to reinforce the edges of scaffold 4, for instance during peripheral fixation of the implant to soft tissue.

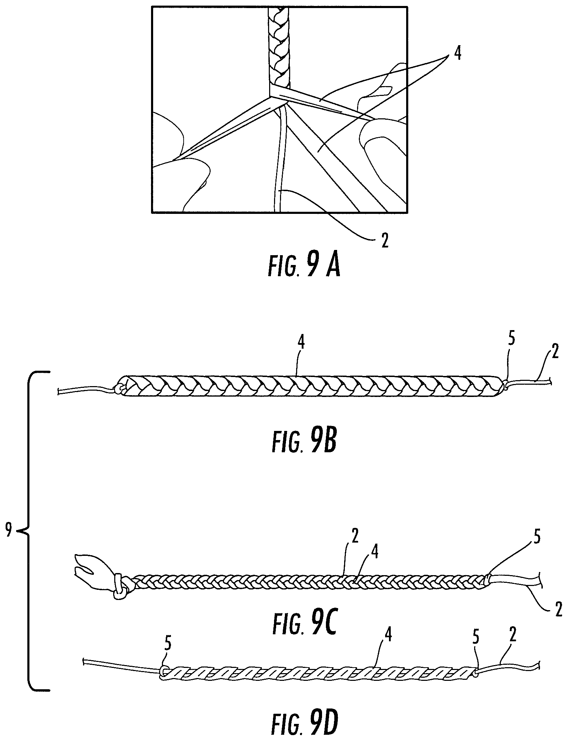

FIGS. 9A-9D illustrate reference examples of composite implants having a more elongated geometry as compared to some of the previously illustrated embodiments. For example, FIG. 9A illustrates a method of forming the implant of FIG. 9B. According to this reference example, a length of suture 2 is held under tension while a plurality of scaffold materials 4 formed into strips are twisted or braided around the suture 2. For instance, three or more strips of scaffold, which can be the same or different, as desired, can be braided around a length of suture 2 held under tension. The suture 2 can then be affixed to the scaffolds at either end of the braid with a locking stitch 5, as shown, though the addition of a locking stitch is not a requirement of the composites.

FIG. 9C illustrates a braided composite implant including a length of suture 2 braided together with two lengths of scaffold 4, and FIG. 9D illustrates a composite including a single length of scaffold 4 wrapped around a single length of suture 2. In both cases, suture 2 is affixed to the scaffolds 4 such that the two are held in contact along a length of the scaffold. In one embodiment, a braided or twisted composite implant can include a reinforcement material (e.g., similar to those shown in FIGS. 3A and 3B) at one or both ends of the composite.

Braided or twisted formations can be made with any design, any number of strands, with any type of twisting or braiding combination, made from any length strip, from straight, U-shaped, or any shaped strips, with any radii of curvature, etc.

As previously mentioned, the utilization of a flexible, pliable elongated members such as suture is not a requirement of the disclosed composites. For instance, in one embodiment a relatively inflexible or non-pliable elongated member, for instance a stiffer metallic or polymeric member, can be utilized to provide the composite with a desired shape. According to one such embodiment, a plurality of scaffold strips can be braided around a preformed, curved and generally non-pliable member so as to provide the finished composite with the desired shape. A relatively inflexible elongated member can extend from a surface of a scaffold or can be attached to a second material that can extend from a surface of the scaffold to aid in manipulation of the composite. For instance, suture can be affixed to either end of an inflexible elongated member so as to form a composite elongated member that can be affixed to a scaffold.

In another embodiment, an elongated member can have a toroid shape and can be provided as an endless loop of material that can be affixed to one or more scaffolds in any suitable fashion, for instance in an open, circular shape or pulled taut, with a closed ovoid shape so as to provide a loop of the elongated member extending from a surface of a scaffold.

As discussed previously, a pliable elongated member of a composite implant as disclosed herein is not limited to suture materials. For example, in one embodiment, a composite implant can include an implantable tendon or ligament as an elongated member of the structure. For instance, synthetic or natural tendons or ligaments as may be used in a transplant procedure, e.g., patellar tendon, hamstring tendon such as semitendinosus tendon and gracilis tendon, anterior tibialis tendon, Achilles tendon, etc., can be utilized in any of the above-described embodiments in place of or in addition to suture materials.

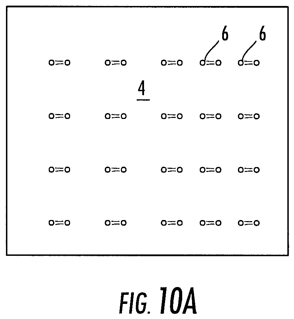

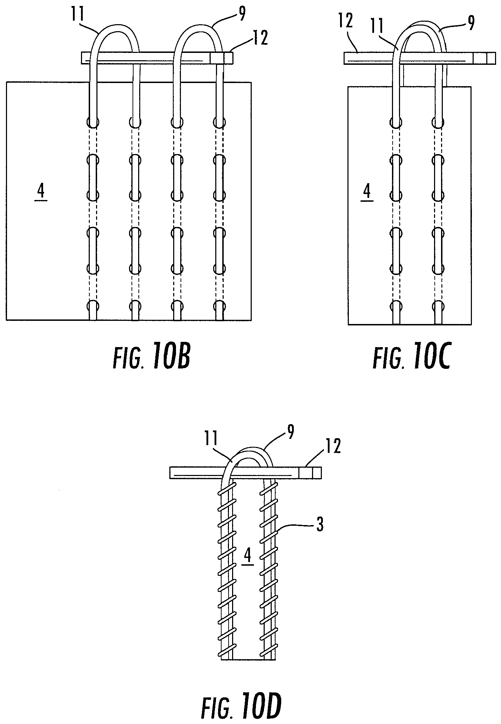

Figs. 10A - 10D illustrate one embodiment of a method for forming a composite implant including implantable tendon as an elongated member. Referring to Fig. 10A, a scaffold 4, e.g., implantable, crosslinked, equine pericardium, can be formed to a desired shape and formed to include a plurality of fenestrations 6. Following formation of the scaffold 4, and with reference to Fig. 10B, a first tendon 9 and a second tendon 11 can be woven through the fenestrations 6 to form a composite implant.

A composite implant can be combined with other implantable devices. For instance, and with reference to FIG. 10B, a composite implant can be combined with a graft harness 12 of a fixation device. Many different types and styles of fixation devices are known in the art, and as such are not described at length herein. For instance, fixation devices as are known in the art and suitable for use with disclosed composite implants can include, without limitation, the ConMed® Linvatec® fixation systems such as the ConMed® Linvatec® Endopearl® system, The Cayenne® AperFix™ system the Arthrotek® EZLoc™ Femoral Fixation Device, the RIGIDfix®) ACL Cross Pin System, and the Stratis Femoral Fixation implant.

The scaffold 4 can then be rolled or folded, as illustrated at FIG. 10C, to the desired size and the formed implant can be fixed with a series of whip stitches with a suture 3, as shown at FIG. 10D, or according to any other suitable fixation process. As can be seen, the two elongated members 9, 11, can extend from the scaffold and can be utilized to locate and fix the implant in place during a surgical procedure and thereby protect the scaffold from damage during the implantation as well as following implantation.

Composite implants can include other components, in addition to a scaffold and an elongated member. For instance, a composite can include reinforcement material such as suture, fibrous mesh (as illustrated in FIGS. 3A and 3B), or the like at the interface between a scaffold and an elongated member and/or along an edge of a scaffold. In one embodiment, a composite implant can include additional functional materials in cooperation with the other components. For instance, a composite implant can include an additional device component such as a portion of a replacement joint, anchoring device, or the like in conjunction with the scaffold and the elongated member affixed thereto.

Disclosed composite implants can be utilized in repair of soft tissue damaged as a consequence of injury, degradation, or disease. For example, composite materials as disclosed herein can be beneficially utilized in surgical procedures including, without limitation, ACL, PCL, MCL, or LCL repair; rotator cuff repair, foot and ankle repair, and the like.

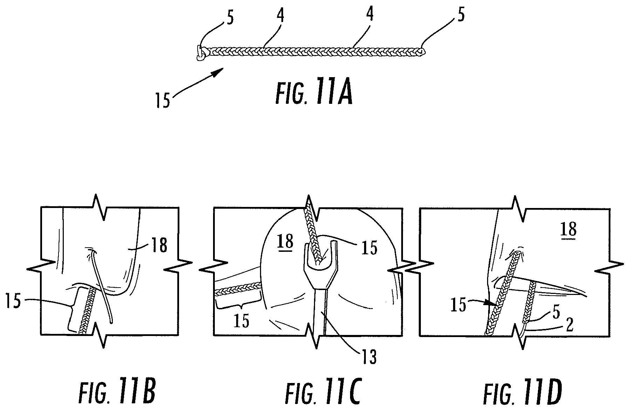

A reference example of a method for utilizing a composite implant is illustrated in FIG. 11. FIG. 11A illustrates a composite including a plurality of scaffold strips 4 braided around a length of suture 2. The suture 2 is knotted with a locking stitch 5 at each end of the braid such that the composite and the suture are coaxial, ensuring that the scaffold will align with the path of the suture during a surgical procedure. At FIG. 11B, a first end of suture is passed through a damaged tendon 18. The implant is then pulled partially through the tendon 18 as pressure is applied to surrounding tissues at 13 (FIG. 11C). The implant 15 is positioned for fixation at FIG. 11D by pulling the implant 15 to the desired location, which includes the placement of scaffold 4 within the damaged tendon, as shown. Pressure can also be applied to the implant via the suture 2 such that the implant 15 is held at the damaged site with a desired tension. Lengths of suture 2 extending from scaffold 4 can be utilized to fix the implant 15 to surrounding tissues (e.g., tendon, ligament, muscle, bone, etc.) following placement of the implant 15. Additional fixation with suture, bioadhesives, etc., may be carried out as necessary with less likelihood of error as compared to previous repair methods, as the implant 15 can be securely held at the desired placement location via the suture 2 of the implant during any additional fixation processes. Thus, a scaffold 4 can be quickly delivered to the desired location with less likelihood of placement error as compared to previous repair methods.

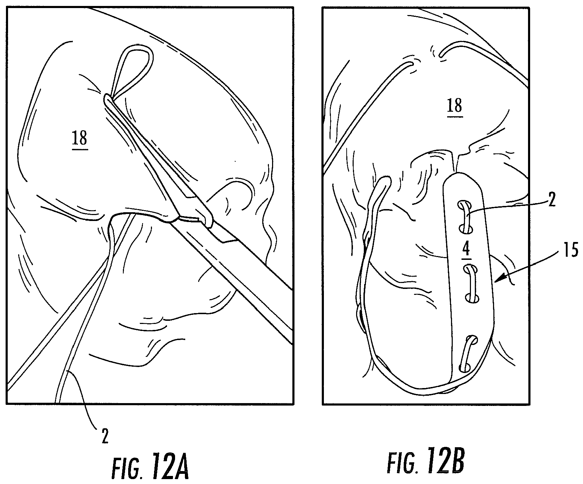

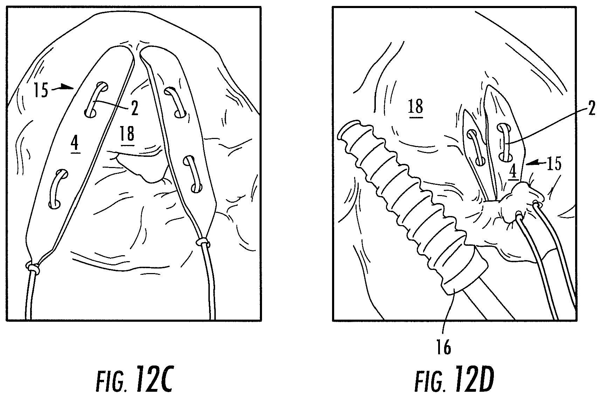

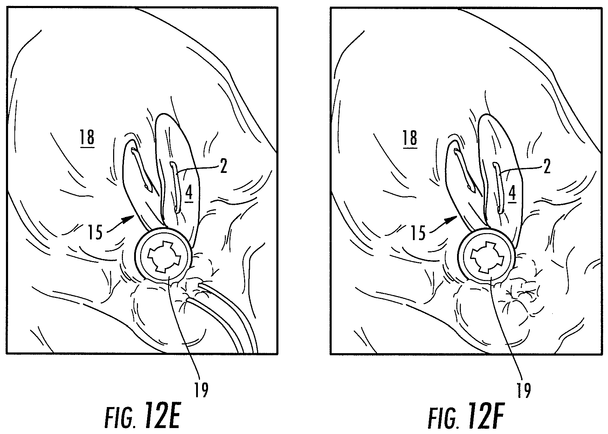

A method of delivering an implant of an embodiment as disclosed herein is illustrated in FIG. 12. In the illustrated embodiment, an implant is being delivered to a tissue 18, which in this particular embodiment, is a torn rotator cuff.

As can be seen at FIG. 12A, an extension of the suture 2 that extends from a surface of the scaffold 4 of the implant 15 is first passed through tissue 18 surrounding the damage. Through application of force on the suture 2 of the implant 15, implant 15 is pulled into place (FIG. 12B and FIG. 12C). At FIG. 12C, the implant 15 is manipulated via tension on the suture 2 of the implant 15 until the scaffold 4 is at the desired location. At FIG. 12D, a tensioning device 16 can be used to return the damaged tissue 18 to the desired footprint. Additional tension as necessary can be applied to the implant 15 with application of force to the suture 2, while mechanically isolating the scaffold 4 from excessive force and thereby preventing damage to the scaffold 4 during and following the procedure. At FIG. 12E, an interference screw 19 is shown in conjunction with the implant 15 to hold the tissue 18. Any extending suture 2 ends can then be trimmed as necessary, as shown at FIG. 12F.

Substantially all longitudinal load placed on an implant during the placement procedure can be borne by the suture, preventing damage to the scaffold strips. Following the procedure, the suture can provide immediate mechanical stabilization of the repair site and can prevent excessive load application to the scaffold while the scaffold can provide a framework and support structure for long term regeneration and repair of surrounding tissue while benefiting from a load distribution effect due to the presence of the suture.

A scaffold can also act as a bolster for disclosed implants. For instance, the presence of a scaffold in conjunction with a suture can prevent damage to surrounding tissue that has been known to develop when sutures have been used exclusively in tissue repair. The scaffold can also minimize the tendency for suture alone to cut out of the host tissue. More specifically, a scaffold can 'cushion' the impact between a suture and surrounding tissue and thereby prevent damage to tissue that can be caused by a fixed suture. In addition, the presence of a scaffold in conjunction with a suture can improve the stability of an implant following fixation at a repair site. In particular, composite implants as disclosed herein are less likely to separate from surrounding tissue following fixation. Thus, implants as disclosed herein can, in one embodiment, provide improved adherence to surrounding tissue following fixation thereto without causing further damage to the surrounding tissue. Moreover, disclosed implants can do so while encouraging long term repair of the damaged tissue.

Of course, disclosed composite implants are not limited to utilization in tendon and ligament repair. Disclosed materials can be utilized in, e.g., repair of soft tissue defects as in cosmetic and plastic reconstructive surgical procedures. Disclosed implants can be utilized to provide support to an elongated member or to prevent damage to surrounding tissue by the elongated member of the composite. For instance, disclosed composites can be used as suture bolsters for damaged tissue in need thereof. Composite materials as disclosed herein can also be useful in supporting damaged tissue, for example as a composite support structure for supporting bladder or urethra tissue, for instance in the treatment of incontinence.

Disclosed implants can also be utilized in repair of tissue other than soft tissue. For instance, in one embodiment, disclosed composite implants can be applied to bone in reconstruction or stabilization of a bone or a joint. Disclosed processes are provided as examples only, however, and composite implants as disclosed herein are not intended to be limited to any particular application. For example, disclosed composites can be utilized in repair of human or animal tissue and in one preferred embodiment, any human or animal soft tissue.

The disclosed composite implants can be utilized to provide both short term and long term repair mechanisms to damaged tissue in a single procedure. This can not only reduce surgery time, as separate tissue augmentation processes need not be required in a reconstructive surgery when utilizing disclosed implants, but can also lead to faster recovery time for patients and more complete repair of damaged tissues.

The disclosed subject matter may be further elucidated with reference to the Example, set for below. The example is provided by way of explanation of the subject matter, not as limitation thereof.

### Example

Starting scaffold material was equine pericardium. The scaffold material was sonicated in a solution of sodium dodecyl sulfate (SDS) in water to remove cellular components. Following sonication, the scaffold material was rinsed three times in a saline rinse and crosslinked according to methods described in U.S. Pat. Nos. 5,447,536 and 5,733,339 to Girardot, et al.. Specifically, the scaffold material was processed with EDC, S-NHS, water, HEPES, hexane diamine, and HCI for 48 hours, followed by another three saline rinses. Following initial preparation, scaffold material was laser cut into straight strips 6mm in width and 20 cm in length, tapered at each end, with holes cut along the center of the implant to allow accurate suture weaving. Throughout this Example, suture material was non-absorbable #2 polyethylene suture.

Using a free needle the suture was woven into the scaffold. A first single knot was formed at each end followed by a second locking knot tied into the first knot to secure it from sliding, thereby protecting the suture/scaffold interface. The locking knots ensured that when a tensile load was applied to the construct that the load was borne primarily be the suture, thereby protecting the collagen scaffold from excessive forces.

The resultant device possessed the mechanical function of the suture and the biological advantage of a tissue augmentation scaffold.

It will be appreciated that the foregoing examples, given for purposes of illustration, are not to be construed as limiting the scope of this disclosure. Further, it is recognized that many embodiments may be conceived that do not achieve all of the advantages of some embodiments, yet the absence of a particular advantage shall not be construed to necessarily mean that such an embodiment is outside the scope of the present disclosure.

## Claims

1. A biocompatible composite implant for soft tissue repair comprising:
a scaffold (4) that allows cellular ingrowth thereto; and
an elongated member affixed to each end of the scaffold (4) with a locking stitch;
wherein the scaffold (4) and the elongated member are held in contact with one another along a length of the scaffold (4), the elongated member extends from a surface of the scaffold (4), and the elongated member is woven through the scaffold (4), **characterised in that** the scaffold (4) comprises collagen.

2. The biocompatible implant of claim 1, wherein the elongated member has a tensile strength greater than that of the scaffold (4).

3. The biocompatible implant of any of the preceding claims, wherein the scaffold (4) comprises crosslinked collagen, preferably, wherein the crosslinked collagen-containing scaffold (4) is a non-glutaraldehyde processed collagen-containing scaffold (4).

4. The biocompatible implant of claim 3, wherein the scaffold (4) comprises crosslinked reconstituted collagen.

5. The biocompatible implant of any of the preceding claims, wherein the elongated member is suture (2).

6. The biocompatible implant of any of claims 1-4, wherein the elongated member is derived from a natural tissue; or wherein the elongated member comprises a woven fabric, preferably wherein the woven fabric covers the entire surface area of the scaffold.

7. The biocompatible implant of any of the preceding claims, wherein the implant has an aspect ratio of about 1.

8. The biocompatible implant of any of the preceding claims, wherein the implant is sterile; or wherein the implant further comprises a reinforcement material covering at least a portion of the surface area of the scaffold (4), preferably wherein the reinforcement material comprises a fabric; or wherein the implant further comprises a biologically active agent incorporated in or on the implant.

9. A method of forming the biocompatible composite implant of any of the preceding claims, the method comprising:
applying an elongated member to a scaffold (4) comprising collagen that allows cellular ingrowth thereto such that the elongated member and the scaffold (4) are held in contact along a length of the scaffold (4); and
affixing the elongated member to each end of the scaffold (4) with a locking stitch such that at least one portion of the elongated member extends from the scaffold (4), wherein the elongated member is woven through the scaffold (4).

10. The method according to claim 9, wherein the elongated member is affixed to the scaffold (4) by use of a bioadhesive; or wherein the method further comprises forming the scaffold (4) to a desired shape; or wherein the method further comprises forming a plurality of fenestrations or perforations (6) in the scaffold (4) at predetermined positions.

## Patentansprüche

1. Biokompatibles Verbundimplantat zur Reparatur von Weichgewebe, umfassend:
ein Gerüst (4), das das Einwachsen von Zellen in das Gerüst ermöglicht; und
ein langgestrecktes Element, das an jedem Ende des Gerüsts (4) mit einem Sicherungsstich befestigt ist;
wobei das Gerüst (4) und das langgestreckte Element entlang einer Länge des Gerüsts (4) miteinander in Kontakt gehalten sind, das langgestreckte Element sich von einer Oberfläche des Gerüsts (4) erstreckt und das langgestreckte Element durch das Gerüst (4) gewebt ist, **dadurch gekennzeichnet, dass** das Gerüst (4) Kollagen umfasst.

2. Biokompatibles Implantat nach Anspruch 1, bei dem das langgestreckte Element eine höhere Zugfestigkeit aufweist als das Gerüst (4).

3. Biokompatibles Implantat nach einem der vorhergehenden Ansprüche, bei dem das Gerüst (4) vernetztes Kollagen umfasst, wobei vorzugsweise das vernetzte kollagenhaltige Gerüst (4) ein nicht mit Glutaraldehyd verarbeitetes kollagenhaltiges Gerüst (4) ist.

4. Biokompatibles Implantat nach Anspruch 3, bei dem das Gerüst (4) vernetztes rekonstituiertes Kollagen umfasst.

5. Biokompatibles Implantat nach einem der vorhergehenden Ansprüche, bei dem das langgestreckte Element eine Naht (2) ist.

6. Biokompatibles Implantat nach einem der Ansprüche 1-4, bei dem das langgestreckte Element von einem natürlichen Gewebe abgeleitet ist; oder bei dem das langgestreckte Element ein textiles Gewebe umfasst, vorzugsweise bei dem das textile Gewebe die gesamte Oberfläche des Gerüsts bedeckt.

7. Biokompatibles Implantat nach einem der vorstehenden Ansprüche, bei dem das Implantat ein Seitenverhältnis von etwa 1 aufweist.

8. Biokompatibles Implantat nach einem der vorhergehenden Ansprüche, bei dem das Implantat steril ist; oder bei dem das Implantat ferner ein Verstärkungsmaterial umfasst, das mindestens einen Teil der Oberfläche des Gerüsts (4) bedeckt, vorzugsweise wobei das Verstärkungsmaterial ein textiles Gewebe umfasst; oder bei dem das Implantat ferner ein biologisch aktives Mittel umfasst, das in oder auf das Implantat integriert ist.

9. Verfahren zum Bilden des biokompatiblen Verbundimplantats nach einem der vorhergehenden Ansprüche, wobei das Verfahren umfasst:
Aufbringen eines langgestreckten Elements auf ein kollagenhaltiges Gerüst (4), das ein zelluläres Einwachsen in dieses ermöglicht, so dass das langgestreckte Element und das Gerüst (4) entlang einer Länge des Gerüsts (4) in Kontakt gehalten werden; und
Befestigen des langgestreckten Elements an jedem Ende des Gerüsts (4) mit einem Sicherungsstich, so dass sich mindestens ein Abschnitt des langgestreckten Elements von dem Gerüst (4) erstreckt, wobei das langgestreckte Element durch das Gerüst (4) hindurchgewebt ist.

10. Verfahren nach Anspruch 9, bei dem das längliche Element unter Verwendung eines Bioklebers am Gerüst (4) befestigt wird; oder bei dem das Verfahren ferner das Bilden des Gerüsts (4) zu einer gewünschten Form umfasst; oder bei dem das Verfahren ferner das Bilden einer Mehrzahl von Fensterungen oder Perforationen (6) in dem Gerüst (4) an vorbestimmten Positionen umfasst.

## Revendications

1. Implant composite biocompatible pour une réparation de tissu mous comprenant :
un échafaudage (4) permettant une croissance cellulaire interne sur celui-ci ; et
un élément allongé fixé à chaque extrémité de l'échafaudage (4) avec un point de verrouillage ;
dans lequel l'échafaudage (4) et l'élément allongé sont maintenus en contact l'un avec l'autre le long d'une longueur de l'échafaudage (4), l'élément allongé s'étend depuis une surface de l'échafaudage (4), et l'élément allongé est tissé à travers l'échafaudage (4), **caractérisé en ce que** l'échafaudage (4) comprend du collagène.

2. Implant biocompatible de la revendication 1, dans lequel l'élément allongé a une résistance à la traction supérieure à celle de l'échafaudage (4).

3. Implant biocompatible selon l'une quelconque des revendications précédentes, dans lequel l'échafaudage (4) comprend du collagène réticulé, de préférence, dans lequel l'échafaudage contenant du collagène réticulé (4) est un échafaudage contenant du collagène traité non glutaraldéhyde (4).

4. Implant biocompatible selon la revendication 3, dans lequel l'échafaudage (4) comprend du collagène reconstitué réticulé.

5. Implant biocompatible selon l'une quelconque des revendications précédentes, dans lequel l'élément allongé est une suture (2).

6. Implant biocompatible selon l'une quelconque des revendications 1 à 4, dans lequel l'élément allongé est dérivé d'un tissu naturel ; ou
dans lequel l'élément allongé comprend un tissu tissé, de préférence dans lequel le tissu tissé recouvre toute la surface superficielle de l'échafaudage.

7. Implant biocompatible selon l'une quelconque des revendications précédentes, dans lequel l'implant a un rapport de forme d'environ 1.

8. Implant biocompatible de l'une quelconque des revendications précédentes, dans lequel l'implant est stérile ; ou dans lequel l'implant comprend en outre un matériau de renforcement recouvrant au moins une partie de la surface superficielle de l'échafaudage (4), de préférence dans lequel le matériau de renforcement comprend un tissu ; ou dans lequel l'implant comprend en outre un agent biologiquement actif incorporé dans ou sur l'implant.

9. Procédé de formation de l'implant composite biocompatible de l'une quelconque des revendications précédentes, le procédé comprenant les étapes consistant à :
appliquer un élément allongé sur un échafaudage (4) comprenant du collagène permettant une croissance cellulaire interne sur celui-ci de telle sorte que l'élément allongé et l'échafaudage (4) sont maintenus en contact sur une longueur de l'échafaudage (4) ; et
fixer l'élément allongé à chaque extrémité de l'échafaudage (4) avec un point de verrouillage de sorte qu'au moins une partie de l'élément allongé s'étend à partir de l'échafaudage (4), dans lequel l'élément allongé est tissé à travers l'échafaudage (4).

10. Procédé selon la revendication 9, dans lequel l'élément allongé est fixé à l'échafaudage (4) à l'aide d'un bioadhésif ; ou
dans lequel le procédé comprend en outre la formation de l'échafaudage (4) à une forme souhaitée ; ou dans lequel le procédé comprend en outre la formation d'une pluralité de fenêtres ou de perforations (6) dans l'échafaudage (4) dans des positions prédéterminées.
